# FASCICULE DE BREVET EUROPEEN

(11) **EP 4 574 178 B1**
(45) Date de publication et mention de la délivrance du brevet: **21.10.2026**
(21) Numéro de dépôt: 24221924.4
(22) Date de dépôt: 19.12.2024
(51) Int. Cl.: A61L 27/18, A61L 27/58, C08L 67/04, A61F 2/00

(54) **PROCEDE POUR INDUIRE UNE AMELIORATION DE LA RESISTANCE MECANIQUE D'UN DISPOSITIF IMPLANTABLE COMPRENANT UN OU DES FIL(S) EN PLA AU TERME D'UNE HYDROLYSE DETERMINEE, ET DISPOSITIF IMPLANTABLE SUSCEPTIBLE D'ETRE OBTENU PAR LEDIT PROCEDE**
VERFAHREN ZUR INDUZIERUNG EINER MECHANISCHEN FESTIGKEIT EINER IMPLANTIERBAREN VORRICHTUNG MIT EINEM ODER MEHREREN DRÄHTEN BEI BESTIMMTER HYDROLYSE UND DAMIT HERGESTELLTE IMPLANTIERBARE VORRICHTUNG
METHOD FOR INDUCING AN IMPROVED MECHANICAL STRENGTH OF AN IMPLANTABLE DEVICE INCLUDING PLA WIRE(S) FOR A DETERMINED HYDROLYSIS AND IMPLANTABLE DEVICE OBTAINABLE THEREBY

(30) Priorité: 21.12.2023 FR 2314790
(43) Date de publication de la demande: 25.06.2025
(73) Titulaire: Cousin Biotech, 59117 Wervicq Sud (FR)
(72) Inventeur: SOLECKI, Gilles, 59117 WERVICQ SUD (FR); OUERGHEMMI, Safa, 59117 WERVICQ SUD (FR)
(74) Mandataire: Cabinet Beau de Loménie

(56) Documents cités:
- WO-A1-2021/042044
- FR-A1- 3 093 911
- US-A1- 2004 234 576
- US-A1- 2013 144 376
- US-A1- 2013 267 137
- US-B2- 10 632 233

## Description

### Domaine Technique

La présente invention concerne le domaine technique des dispositifs implantables au moins en partie résorbables et au moins en partie textiles, en particulier pour le traitement des hernies, ayant une résistance mécanique améliorée au terme d'une période d'hydrolyse prolongée déterminée.

La présente invention concerne également le domaine technique des procédés permettant d'améliorer la résistance mécanique au terme d'une période d'hydrolyse prolongée des dispositifs implantables au moins en partie textiles et au moins en partie résorbables, notamment pour le traitement des hernies abdominales.

### Technique antérieure

Il est connu d'utiliser des dispositifs implantables au moins en partie textiles et au moins en partie résorbables ou non résorbables pour le traitement des hernies abdominales.

Ces dispositifs implantables comprennent généralement un panneau textile implantable, au moins en partie résorbable ou non résorbable, sensiblement plan et/ou comprenant une forme anatomique en trois dimensions pour combler un defect pariétal. Le panneau textile est généralement un tricot ou un tissu, par exemple un tricot en fils monofilamentaires apportant suffisamment de résistance mécanique, en polypropylène ou en polyéthylène par exemple lorsque non résorbable ou en P4HB lorsque résorbable par exemple. Lorsque le panneau textile de renfort est non résorbable, il est destiné à rester implanté chez le patient en traitement de la hernie. Lorsque le panneau textile de renfort est résorbable, il est destiné à être hydrolysé par le milieu d'implantation naturel du patient qui est un milieu aqueux et donc à disparaitre au terme d'une période déterminée.

Le choix d'un dispositif implantable résorbable ou non est fonction notamment du type de patient, en particulier si ce dernier présente des risques tels que l'obésité, le diabète, le tabagisme, ou encore présente une combinaison de ces différents risques. Il a été observé que les patients présentant l'un ou plusieurs des facteurs de risques énoncés ci-avant sont davantage sujets aux risques de récidives d'une hernie abdominale, et surtout aux infections au niveau du dispositif implantable. Ces infections lorsqu'elles ne peuvent être traitées par un traitement médicamenteux nécessite une intervention chirurgicale afin de retirer le dispositif implantable. Ces infections surviennent généralement au terme d'une période de plusieurs mois. La pratique chirurgicale dans le domaine du traitement des hernies ventrales tend ainsi à ne plus utiliser de dispositifs implantables en polypropylène ou en polyester, c'est-à-dire non résorbables, pour traiter les patients avec des facteurs de risques d'infections car ces derniers seront amenés à subir une chirurgie corrective consistant à retirer le dispositif implantable. Afin de pallier à cet inconvénient, les patients avec des facteurs de risques d'infection sont traités avec des dispositifs implantables résorbables. On connait ainsi en traitement des hernies abdominales pour ces patients à risques d'infections, le dispositif implantable commercialisé sous la marque PHASIX^{®} avec des fils en P4HB résorbables avec une résorption complète au terme de 18 mois. Ainsi, en cas d'infections au terme de plusieurs mois d'implantation, il n'est pas nécessaire de retirer le dispositif implanté.

Les dispositifs implantables résorbables disponibles sur le marché permettent de traiter relativement correctement l'infection sans avoir à explanter le dispositif implanté (puisqu'il disparait totalement à terme) mais la probabilité de réapparition de la récidive de la hernie est plus importante.

Pour les patients avec des risques d'infections, la majorité des récidives apparait avant la fin de la première année après implantation, c'est-à-dire vers 12 mois. Ces récidives qui interviennent pendant la première année apparaissent en périphérie du dispositif implanté, c'est-à-dire sur des zones anatomiques non couvertes par le dispositif implanté. Au-delà de la première année, les récidives apparaissent sur les zones anatomiques couvertes par le dispositif implanté résorbable, qui se rompt par une perte de résistance due à la dégradation par hydrolyse.

Il est ainsi recherché d'améliorer la résistance mécanique des dispositifs implantés résorbables en-cours d'hydrolyse, et ce plusieurs mois après le début de l'hydrolyse, afin d'éviter une récidive de la hernie.

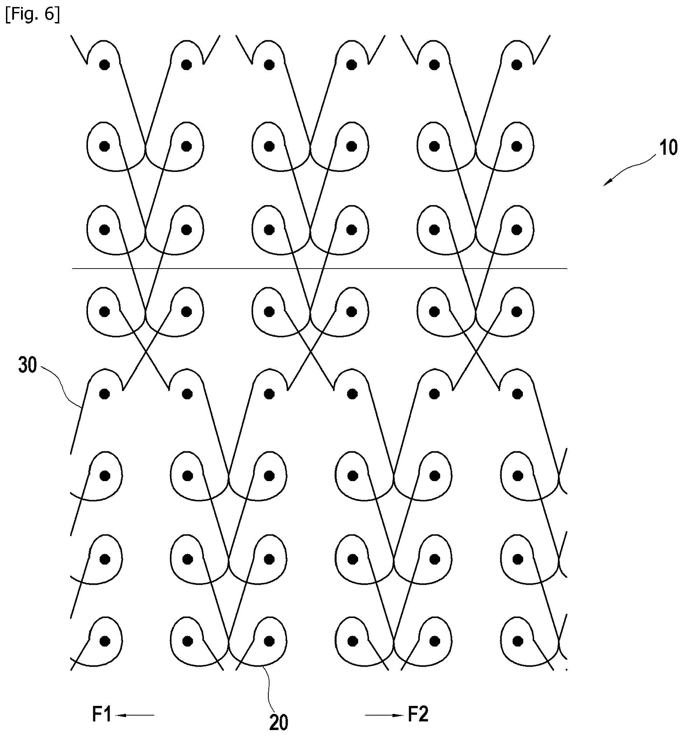

La publication intitulée « In Vitro Degradation of Poly(L-lactic acid) fibers produced by melt spinning » ; A.Pegoretti, L.Fambri, C.Migliaresi, Department of Materials Engineering, University of Trento, via Mesiano 77, 38050 Trento, Italy, met en évidence que les propriétés mécaniques des fibres de plus faibles diamètres (environ 72 µm) se dégradent plus vite que celles de fibres dont le diamètre est plus élevée (environ 120 µm) sous l'effet de l'hydrolyse du PLLA. Cette étude met en évidence que le taux de dégradation dépendrait de nombreux facteurs, dont le type de polymère, des conditions de fabrication du PLLA (par la voie fondue ou solvantée) et le milieu de dégradation. En particulier, plus le ratio surface/volume est faible et plus la masse molaire initiale est élevée, plus les taux de dégradation de la masse molaire et des propriétés mécaniques sont ralenties. On remarque qu'au terme de 16 semaines (4 mois), la résistance à rupture (MPa), relevée sur la figure 6, chute de 900 MPa à environ 225 MPa pour un monofilament de diamètre 120 microns, soit une perte de résistance mécanique de l'ordre de 75 % pour une durée d'hydrolyse de 4 mois. Pour des fibres ayant un diamètre de 72 microns, la résistance à rupture chute de 975 MPa à 110 MPa après 4 mois d'hydrolyse (soit une perte de la résistance mécanique de 89 %, cf. figure 7b). Les fibres de 120 µm conservent une résistance à rupture supérieure à celle des fibres de 72 µm mais la dégradation de la résistance à rupture est supérieure ou égale à 75 % dans les deux cas après une durée d'hydrolyse de 4 mois dans une solution tamponnée à pH 7.4 et à une température de 37°C.

L'homme du métier est ainsi incité à augmenter le diamètre des filaments des dispositifs implantables afin de retarder la perte de résistance mécanique, néanmoins on observe une perte de résistance mécanique trop importante (70 %) au terme de 4 mois. Or, il est recherché une résistance mécanique qui ne soit pas trop abaissée au terme de 4 mois, voire encore significative au terme de 5 mois, et au terme de 8 mois, puisque les risques d'infections et de récidive sont plus importants vers 12 mois.

De plus, il n'est pas possible de mettre en œuvre des filaments ayant un diamètre trop important car le dispositif implantable doit rester souple, en particulier suffisamment pour pouvoir être enroulé sur lui-même et disposé dans un trocart d'insertion de quelques mm de diamètre puis être déroulé sur le site d'implantation.

On pourrait ainsi ajouter un revêtement résorbable sur les filaments eux-mêmes résorbables afin de retarder l'hydrolyse desdits fils, et différer la perte de résistance mécanique. Néanmoins, cette disposition nécessite une étape de revêtement des fils, et à tendance à rigidifier les fils, ce qui est au détriment du confort du patient, et abaisse la souplesse du dispositif implantable.

WO 2021/042044 décrit un dispositif implantable, pouvant être utilisé pour le traitement de la hernie, comprenant du poly-4-hydroxybutyrate (P4HB), cherchant à optimiser sa dégradation pour éviter une seconde intervention chirurgicale.

La présente invention a ainsi pour objet un procédé permettant d'induire une amélioration de la résistance mécanique à l'hydrolyse, en particulier en milieu aqueux, d'un dispositif implantable comprenant un ou des fil(s) monofilamentaire(s) en (co)polymère(s) d'acide lactique, et ce sans perte de la souplesse dudit dispositif implantable afin qu'il puisse être inséré dans un trocart.

La présente invention a également pour objet un dispositif implantable en tout ou partie résorbable, comprenant un ou des fil(s) en (co)polymère d'acide lactique, et ayant une résistance mécanique à l'hydrolyse, en particulier en milieu aqueux, améliorée, en particulier à 5 mois et/ou à 8 mois.

### Exposé de l'invention

La présente invention répond aux problèmes précités en ce qu'elle a pour objet, selon un premier aspect, un procédé pour induire une amélioration de la résistance mécanique (N/cm) d'un dispositif implantable après une période déterminée d'hydrolyse dudit dispositif implantable, en particulier mesurée en semaines ou en mois, ledit dispositif implantable comprenant un ou des fil(s) comprenant au moins un (co)polymère d'acide lactique, ledit procédé comprenant en outre , lesdites étapes ayant lieu avantageusement dans cet ordre: i)- une étape de fourniture d'un dispositif implantable comprenant un ou des fil(s) comprenant au moins un (co)polymère d'acide lactique; ii)- une étape de traitement dudit dispositif implantable avec du dioxyde de carbone (CO₂) supercritique ; iii)- une étape d'obtention d'un dispositif implantable ayant une résistance mécanique supérieure ou égale à 10 N/cm, ou à 15N/cm, ou à 20N/cm, ou à 22N/cm, de préférence supérieure ou égale à 25 N/cm ou à 27 N/cm ou à 29N/cm, après 20 semaines d'hydrolyse, en particulier dans un milieu aqueux.

Il a été découvert de manière surprenante que le traitement par du CO₂ sc d'un dispositif implantable au moins en partie textile et au moins en partie résorbable, avec des fils en (co)polymère d'acide lactique permet d'améliorer la résistance mécanique de ce dispositif implantable au-cours de son hydrolyse.

Ainsi, il a été observé qu'un dispositif implantable au moins en partie textile comprenant des fils monofilamentaires en (co)polymère d'acide lactique traité par CO₂ sc a une résistance mécanique (N/cm) multiplié par trois au moins à 5 mois d'hydrolyse comparativement à un dispositif implantable identique non traité par CO₂ sc.

### Dispositif implantable

De préférence, le dispositif implantable comprend, notamment est, une plaque textile plane ou en trois dimensions ou comprenant une partie plane et une partie en trois dimensions.

On comprend dans le présent texte par plaque textile plane ou partie plane que cette partie ou cette plaque s'étend dans un plan en deux dimensions, selon des axes x et y, l'épaisseur de la plaque ou de la partie étant non significative et/ou non considérée. On comprend dans le présent texte par plaque textile en trois dimensions ou partie en trois dimensions que cette partie ou cette plaque s'étend selon trois dimensions, selon des axes x et y et z, l'épaisseur de la plaque ou de la partie étant mesurée selon l'axe z.

De préférence, le dispositif implantable comprend un ou plusieurs textile(s), notamment un ou des panneaux(x) textile(s), tricoté(s) et/ou tissé(s) et/ou tressé(s), encore de préférence tricoté(s).

De préférence, le dispositif implantable, en particulier le textile ou le panneau textile, a une masse surfacique supérieure ou égale à 10 g/m², ou à 20 g/m² ou à 30 g/m² ou à 40g/m².

Encore de préférence, le dispositif implantable, en particulier le textile ou le panneau textile, a une masse surfacique supérieure ou égale à 50 g/m², ou à 60 g/m² ou 70 g/m² ou à 80 g/m² ou à 90 g/m² ou à 100 g/m², préférentiellement supérieure ou égale à 110 g/m².

De préférence, le dispositif implantable, en particulier le textile ou le panneau textile, a une masse surfacique inférieure ou égale à 300 g/m², ou à 250 g/m² ou à 230 g/m², ou à 220 g/m² ou à 200 g/m², encore de préférence inférieure ou égale à 190 g/m² ou à 180 g/m².

Avantageusement, la plaque textile plane ou en trois dimensions comprend, ou est constituée essentiellement, d'un ou plusieurs textile(s).

Dans un mode de réalisation, le tricot est un tricot à mailles cueillies ou à mailles chaines (i.e. à mailles jetées).

De préférence, le dispositif implantable comprend, ou le textile ou la plaque textile est, un tricot à mailles jetées, encore de préférence ledit tricot à mailles jetées comprend un ou des fil(s) monofilamentaire(s).

De préférence, le dispositif implantable est arrangé pour le traitement d'une hernie abdominale, par exemple ventrale ou inguinale.

Avantageusement, le dispositif implantable est au moins en partie textile.

Avantageusement, le dispositif implantable et/ou le textile et/ou la plaque textile est/sont au moins en partie résorbable, de préférence au moins 50% ou 60% ou 70% ou 80% ou 90% ou 95% ou 99% ou 100% environ en masse du dispositif implantable ou du textile ou de la plaque est résorbable.

On comprend dans le présent texte par résorbable qu'une partie ou la totalité du dispositif implantable et/ou d'un textile et/ou d'une plaque textile est résorbable par hydrolyse, c'est-à-dire qu'elle est dégradée chimiquement et/ou mécaniquement progressivement par hydrolyse dans un milieu aqueux à température ambiante (par exemple à une température supérieure ou égale à 20°C, ou à 37°C) au terme d'une période déterminée et/ou une fois implantée dans un organisme vivant (biorésorbable), en particulier un mammifère, plus particulièrement le corps humain, au terme d'une période déterminée par hydrolyse, par exemple de 4 mois ou 5 mois ou 8 mois ou 10 mois ou 12 mois ou plus.

Un textile peut comprendre un ou plusieurs fil(s) monofilamentaire(s) et/ou un plusieurs fil(s) multifilamentaire(s).

De préférence, le milieu aqueux comprend au moins 50% ou 60% ou 70% ou 80% ou 90% en masse ou en volume d'eau, en particulier d'eau distillée et/ou osmosée.

De préférence, le milieu aqueux a un pH supérieur ou égal 6 et inférieur ou égal à 8, encore de préférence supérieur ou égal à 6.5 ou 6.7 ou 6.8 ou à 7.0 ou à 7.2, préférentiellement inférieur ou égal à 7.8 ou à 7.6.

Par exemple, le milieu aqueux a un pH de l'ordre de 7.4 à +/- 0.1.

De préférence, le milieu aqueux est tamponné avec une solution saline tamponnée au phosphate (PBS).

De préférence, un fil monofilamentaire a un diamètre supérieur ou égal à 0,01mm, encore de préférence supérieur ou égal à 0,05 mm, préférentiellement supérieur ou égal à 0,10 mm.

De préférence, un fil monofilamentaire a un diamètre inférieur ou égal à 3 mm, encore de préférence inférieur ou égal à 2 mm ou à 1 mm ou à 0,80 mm, préférentiellement inférieur ou égal à 0,70 mm ou à 0,60 mm ou à 0,50 mm ou à 0,40 mm ou à 0,30 mm ou à 0,20 mm.

De préférence, le ou les fil(s) monofilamentaire(s) et/ou multifilamentaire(s) comprend/comprennent (chacun), ou est/sont constitué(s) de, un ou plusieurs matériaux (bio)résorbable(s) choisis parmi : un polymère d'acide lactique de forme L ou D ou de formes L et D, un polymère d'acide glycolique, un copolymère d'acide lactique (L et/ou D) et d'acide glycolique, le polycaprolactone, le poly-4-hydroxybutyrate (P4HB), ou un mélange de ces derniers.

La forme L (Lévogyre) et la forme D (Dextrogyre) sont deux isomères de l'acide lactique.

Le polymère d'acide lactique peut être un homopolymère ou un copolymère.

On comprend dans le présent texte par copolymère tout polymère comprenant au moins deux unités de répétition différentes, et éventuellement au moins trois unités de répétition différentes (par exemple un terpolymère).

On comprend par (co)polymère d'acide lactique, un homopolymère d'acide lactique (comprenant des unités de répétition de forme L et/ou de forme D), et tout copolymère comprenant au moins des unités de répétition d'acide lactique (comprenant des unités de répétition de forme L et/ou de forme D) et des unités de répétition différentes de l'acide lactique, par exemple d'acide glycolique.

Dans un mode de réalisation, le ou les fil(s) monofilamentaire a/ont (chacun) un allongement à rupture inférieur ou égal à 100%, en particulier inférieur ou égal à 70%, plus particulièrement inférieur ou égal à 60% ou à 50% ou à 45%.

Avantageusement, le ou les fil(s) monofilamentaire a/ont (chacun) un allongement à rupture supérieur ou égal à 10%, en particulier supérieur ou égal à 15% ou à 20%. Dans un mode de réalisation, le ou les fil(s) monofilamentaire a/ont (chacun) une ténacité (cN/dtex) supérieure ou égale à 1 cN/dtex, de préférence supérieure ou égale à 2 ou 3 cN/dtex.

Avantageusement, le ou les fil(s) monofilamentaire(s) a/ont (chacun) une ténacité (cN/dtex) inférieure ou égale à 30 cN/dtex, de préférence inférieure ou égal à 20 cN/dtex, encore de préférence inférieure ou égale à 15 cN/dtex, en particulier inférieure ou égale à 10 cN/dtex.

Dans un mode de réalisation, le ou les fil(s) monofilamentaire(s) a/ont (chacun) une charge à la rupture supérieure ou égale à 300 cN, de préférence supérieure ou égale à 400 cN ou à 500 cN ou à 600 cN ou à 700 cN ou à 800 cN.

Avantageusement, le ou les fil(s) monofilamentaire(s) a/ont (chacun) une charge à la rupture inférieure ou égale à 3000 cN, de préférence inférieure ou égale à 2000 cN ou à 1500 cN.

La charge à rupture, la ténacité, et l'allongement à rupture du fil monofilamentaire ont été mesurés selon la norme EN 13895 datant de juin 2003, et intitulée « Textiles - Monofilaments- Détermination des propriétés en traction ».

Dans un mode de réalisation, le ou les fil(s) monofilamentaire(s) a/ont (chacun) une masse linéique (dtex) supérieure ou égale à 50 dtex, de préférence supérieure ou égale à 100 dtex ou à 150 dtex ou à 180 dtex ou à 200 dtex.

Avantageusement, le ou les fil(s) monofilamentaire(s) a/ont (chacun) une masse linéique (dtex) inférieure ou égale à 800 dtex, de préférence inférieure ou égale à 700 dtex ou à 600 dtex ou à 500 dtex ou à 400 dtex ou à 300 dtex, par exemple de l'ordre de 220 dtex à +/- 50 dtex.

La masse linéique est mesurée à l'aide de la norme NF EN 13392 datant de septembre 2001, intitulée « Textiles - Monofilaments - Détermination de la masse linéique ». On comprend par un fil monofilamentaire, tout fil comprenant un seul filament, et capable d'être mise en œuvre sur un métier textile, en particulier un métier à tricoter, un métier à tisser, un métier à tresser.

Dans un mode de réalisation, le dispositif implantable comprend un tricot à mailles jetées comprenant des fils monofilamentaires, en particulier :
- un premier fil monofilamentaire forme des mailles tricots ouvertes et/ou fermées, en particulier des mailles atlas, plus particulièrement s'étendant sur au moins trois colonnes de mailles (ou sur au moins trois aiguilles), en particulier sur au plus 20 ou 15 ou 10 colonnes de mailles (ou aiguilles); et
- un second monofilamentaire forme des mailles tricots ouvertes et/ou fermées, en particulier des mailles atlas, plus particulièrement s'étendant sur au moins trois colonnes de mailles (ou sur au moins trois aiguilles), en particulier sur au plus 20 ou 15 ou 10 colonnes de mailles (ou aiguilles),en particulier le second fil est supporté par une barre de guidage travaillant dans une direction opposée à la barre de guidage supportant le premier fil monofilamentaire ; et
- éventuellement un troisième fil monofilamentaire forme des mailles tricots ouvertes et/ou fermées, en particulier des mailles atlas, plus particulièrement s'étendant sur au moins trois colonnes de mailles (ou sur au moins trois aiguilles), en particulier sur au plus 20 ou 15 ou 10 colonnes de mailles (ou aiguilles), en particulier selon le même schéma de mailles que celui du premier fil monofilamentaire ; et
- éventuellement un quatrième fil monofilamentaire forme des mailles tricots ouvertes et/ou fermées, en particulier des mailles atlas, plus particulièrement s'étendant sur au moins trois colonnes de mailles (ou sur au moins trois aiguilles), en particulier sur au plus 20 ou 15 ou 10 colonnes de mailles (ou aiguilles), en particulier le quatrième fil est supporté par une barre de guidage travaillant dans une direction opposée à la barre de guidage supportant le troisième fil monofilamentaire.

Dans un mode de réalisation, le tricot comprend :
- un premier fil monofilamentaire enfilé, en particulier à plein, sur une première barre à passettes B1 selon l'armure suivante décrite conformément à la norme ISO 11676, datant de 2014 : 2- 3/ 2- 1/ 2- 3/ 2- 1/ 1- 0/ 1- 2/ 1- 0/ 1- 2// ;
- un second fil monofilamentaire enfilé, en particulier à plein, sur une seconde barre à passettes B2 selon l'armure suivante décrite conformément à la norme ISO 11676, datant de 2014 : 1- 0/ 1- 2/ 1- 0/ 1- 2/ 2- 3/ 2- 1/ 2- 3/ 2- 1 // ;
- et éventuellement :
   - un troisième fil monofilamentaire enfilé, en particulier à plein, sur une troisième barre à passettes B3 selon l'armure suivante décrite conformément à la norme ISO 11676, datant de 2014 : 2- 3/ 2- 1/ 2- 3/ 2- 1/ 1- 0/ 1- 2/ 1- 0/ 1- 2// ; et/ou
   - un quatrième fil monofilamentaire enfilé, en particulier à plein, sur une quatrième barre à passettes B4 selon l'armure suivante décrite conformément à la norme ISO 11676, datant de 2014 : 1- 0/ 1- 2/ 1- 0/ 1- 2/ 2- 3/ 2- 1/ 2- 3/ 2- 1 //.

De préférence, la barre B1 se déplace/tricote dans une direction opposée à la direction de déplacement/tricotage de la barre B2.

De préférence, la barre B3 se déplace/tricote dans une direction opposée à la direction de déplacement/tricotage de la barre B4.

De préférence, la barre B1, respectivement B2, se déplace/tricote dans la même direction que la direction de déplacement/tricotage de la barre B3, respectivement B4. Avantageusement, travailler avec des barres à passettes ayant le même mouvement permet de tricoter avec un seul fil par barre, et ainsi de mieux équilibrer les tensions exercées sur un fil que si une barre supporte deux fils, tout en augmentant la masse surfacique du tricot.

Par exemple, un tricot tricoté avec deux barres B1 et B2, chaque barre supportant un fil, permet d'obtenir une masse surfacique d'environ 120 g/m² à +/- 20 g/m².

Par exemple, un tricot tricoté avec quatre barres B1, B2, B3 et B4, chaque barre supportant un fil, permet d'obtenir une masse surfacique d'environ 170 g/m² à +/- 20 g/m².

Le dispositif implantable peut comprendre un textile, par exemple un tricot, comprenant un ou plusieurs revêtements polymérique(s) résorbable(s), par exemple à base de polyvinylpyrrolidone, d'un polymère cellulosique, d'un polymère de cyclodextrine, d'un polymère de k-carraghénane, ou une combinaison de ces derniers.

### Amélioration de la résistance mécanique (N/cm)

La résistance mécanique (Newtons/cm), ou valeur d'éclatement (Newtons/cm), est mesurée selon la méthode de test décrite dans la norme ASTM D3787-7 (2011), intitulée « Bursting Strength of Textiles - Constant-Rate of Traverse (CRT) Ball Burst Test » ou « Résistance à l'éclatement des textiles - Essai d'éclatement à la bille à vitesse constante ».

En particulier, cette norme permet de déterminer une résistance à la rupture totale F exprimée en newtons. La résistance à la rupture exprimée en N/cm est calculée en divisant la résistance à la rupture totale exprimée en Newtons par le périmètre de la bille exprimé en cm.

En particulier, le diamètre de la bille, conforme à l'ASTM D3787-7 (2011), est de 2,54 cm (soit « un pouce anglais » ou « pouce technique international ») et a donc un périmètre de 2,54 x Pi en cm. La résistance à la rupture exprimée en N/cm est donc calculée selon la formule : F/(2,54 x Pi).

En particulier, les tests sont effectués sur une moyenne de trois à six échantillons textiles, en particulier six, d'environ 10 cm * 10 cm. Plus particulièrement, l'échantillon (10 cm x 10 cm) est solidement fixé sans tension entre deux plaques circulaires rainurées. Puis, l'accessoire mobile, comprenant une tige ayant une extrémité comprenant une bille, est fixé sur le mors de traction et l'accessoire se déplace vers la surface de l'échantillon textile pour que la bille perfore ledit échantillon textile à une vitesse de déplacement constante de 305 mm/min. Une force est exercée contre la surface de l'échantillon textile par la bille d'acier (le diamètre de la bille est en particulier d'un pouce, soit de 2,54 cm). La valeur d'éclatement par la bille correspond à la force nécessaire pour percer l'échantillon. Cette valeur en (newtons) est divisée par la circonférence de la balle de diamètre un pouce anglais (2,54 x Pi = 7,98 cm environ) pour obtenir la valeur de résistance en newtons par centimètre (N/cm).

Ce test permet avantageusement de mettre en jeu toute la surface textile de l'échantillon testé.

Avantageusement, le dispositif implantable, en particulier la plaque textile (plane ou en trois dimensions) ou le tricot, a une résistance mécanique ou valeur d'éclatement avant implantation ou à 0 jour d'hydrolyse, supérieure ou égale à 16 N/cm pour le traitement d'une hernie inguinale.

Avantageusement, le dispositif implantable, en particulier la plaque textile (i.e. plane ou en trois dimensions) ou le tricot, a une résistance mécanique ou valeur d'éclatement avant implantation ou à 0 jour d'hydrolyse, supérieure ou égale à 32 N/cm pour le traitement d'une hernie ventrale.

De préférence, le dispositif implantable, en particulier la plaque textile (i.e. plane ou en trois dimensions) ou le tricot, a une résistance mécanique (N/cm) de R0 (N/cm) avant implantation ou à 0 jour d'hydrolyse, et une résistance mécanique (N/cm) R1 au terme de 3 mois d'hydrolyse, avec R1 inférieure ou égale à 0,98 x R0 ou à 0,93 x R0 et supérieure ou égale à 0,60 x R0 ou à 0,70 ou à 0,80 x R0.

De préférence, le dispositif implantable a une résistance mécanique (N/cm) de R0 (N/cm) avant implantation ou à 0 jour d'hydrolyse, et une résistance mécanique (N/cm) R2 au terme de 5 mois d'hydrolyse, avec R2 inférieure ou égale à 0,94 x R0 ou à 0,89 x R0 et supérieure ou égale à 0,50 x R0 ou à 0,60 x R0. De préférence, le dispositif implantable a une résistance mécanique (N/cm) de R0 (N/cm) avant implantation ou à 0 jour d'hydrolyse, et une résistance mécanique (N/cm) R3 à 8 mois d'hydrolyse, avec R3 inférieure ou égale à 0,60 ou à 0,50 x R0 et supérieure ou égale à 0,20 ou à 0,30 ou à 0,35 x R0.

De préférence, la baisse de la résistance mécanique observée pour le dispositif implantable traité selon l'invention est comprise entre un minimum de 10 % et un maximum de 27 % au terme de 3 mois d'hydrolyse contre plus de 30 % pour un dispositif implantable de structure identique mais non traité selon l'invention par du CO₂ sc.

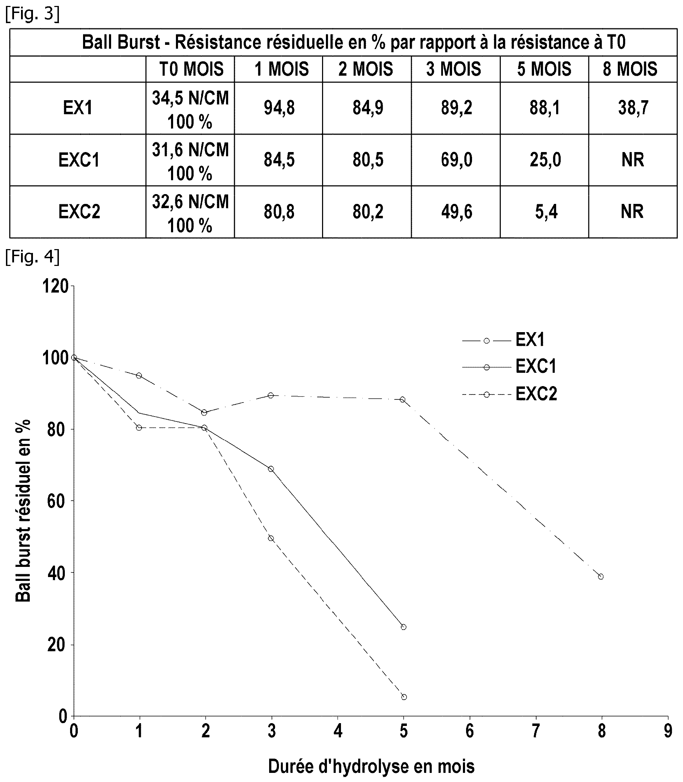

Avantageusement, la baisse de la résistance mécanique (N/cm) observée pour le dispositif implantable traité selon l'invention au CO₂sc, en particulier déduite de la courbe EX1 représentée sur la figure 4 annexée, est d'environ 12 % au terme de 5 mois d'hydrolyse et de 61 % au terme de 8 mois d'hydrolyse contre 95 % au terme de 5 mois d'hydrolyse et 100% au terme de terme de 8 mois d'hydrolyse, en particulier déduite de la courbe EXC2 représentée sur la figure 4 annexée, pour un dispositif implantable de structure identique non traité selon l'invention par du CO₂ sc.

### CO₂ sc, ou dioxyde de carbone supercritique ou dans un état supercritique

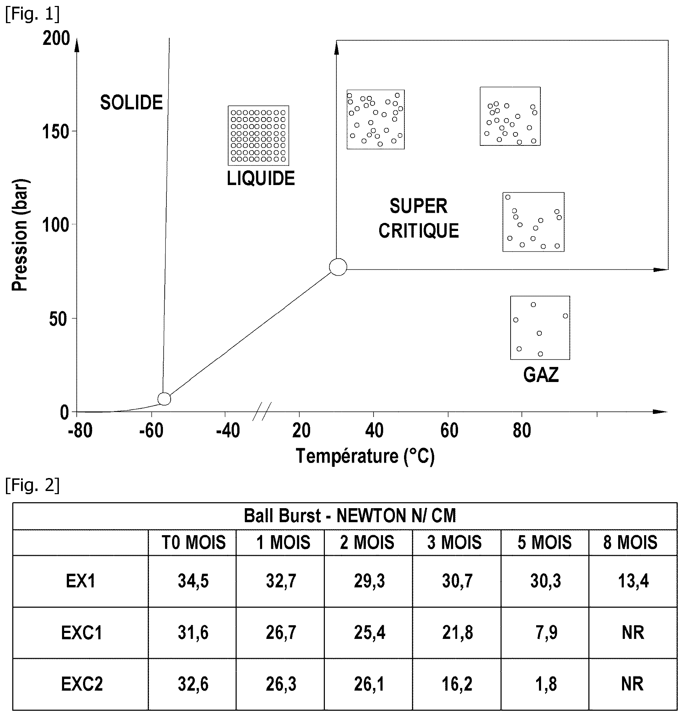

Le dioxyde de carbone pur passe dans un état supercritique lorsqu'il est soumis à une pression supérieure à une pression critique et chauffé au-dessus une température critique, en particulier selon le diagramme de pression (bars) et de température (°C) représentée à la figure 1 annexée.

Avantageusement, la pression critique est supérieure ou égale à 73 bars, et la température critique est supérieure ou égale à 31°C.

Avantageusement, le dioxyde de carbone dans un état supercritique a des propriétés particulières : comparable à celles d'un liquide dont la densité est supérieure ou égale à 0,2 g/cm³ et inférieure ou égale à 1 g/cm³ avec un pouvoir de solvant, et comparable à celles d'un gaz avec une grande diffusivité supérieure ou égale à 10⁻⁴ cm²/seconde, et inférieure ou égale à 10⁻³ cm²/seconde, notamment est miscible avec d'autre gaz, et/ou a une tension superficielle faible.

Avantageusement, le dioxyde de carbone dans un état supercritique (désigné ci-avant et ci- après CO₂ sc) a une viscosité supérieure ou égale à 10 µPa.s et inférieure ou égale à 100 µPa.s.

Dans un mode de réalisation, l'étape de traitement (ii) comprend les étapes suivantes, en particulier ayant lieu dans l'ordre suivant :
- iia) la fourniture de dioxyde de carbone liquide ;
- iib) l'application au dioxyde de carbone de conditions de température et de pression permettant de faire passer le dioxyde de carbone d'un état liquide à un état supercritique ;
- iic) l'alimentation du dioxyde de carbone dans état supercritique à une enceinte, en particulier un autoclave, comprenant le dispositif implantable, en particulier ladite enceinte comprend un volume de traitement hermétique dans lequel les conditions de température et de pression sont déterminées en sorte de maintenir le dioxyde de carbone dans un état supercritique.

Avantageusement, le dioxyde de carbone est stocké dans un container à l'état liquide lors de l'étape iia).

Avantageusement, l'étape iib) comprend tout d'abord le pompage du dioxyde de carbone à l'état liquide et l'application d'une pression déterminée pour la transformation du dioxyde de carbone à l'état liquide dans un état gazeux, puis le dioxyde de carbone à l'état gazeux est chauffé à une température déterminée pour la transformation du dioxyde de carbone à l'état gazeux en du dioxyde de carbone dans un état supercritique.

Avantageusement, l'étape iic) comprend le pompage et l'alimentation du volume de traitement de l'enceinte avec le CO₂ sc.

Avantageusement, après l'étape iic), la pression du CO₂ sc est abaissée, par exemple selon une pression déterminée sur le diagramme de la figure 1, pour atteindre un état gazeux, puis le CO₂ gazeux obtenu subit une étape de séparation permettant de séparer le CO₂ d'éventuel(s) contaminant(s), le CO₂ gazeux résultant est condensé pour atteindre un état liquide, et éventuellement être alimenté au container de stockage (par exemple pour subir un nouveau cycle de traitement selon l'étape ii)). De préférence, à l'étape ii), en particulier à l'étape iib) et/ou iic), le dioxyde de carbone est chauffé, en particulier maintenu à l'étape iic), à une température supérieure ou égale à 10°C, encore de préférence supérieure ou égale à 20°C, préférentiellement supérieure ou égale à 25°C ou à 30°C ou à 33°C ou à 35°C.

De préférence, à l'étape ii), en particulier à l'étape iib) et/ou iic), le dioxyde de carbone est chauffé, en particulier maintenu à l'étape iic), à une température inférieure ou égale à 100°C, encore de préférence inférieure ou égale à 80°C, préférentiellement inférieure ou égale à 70°C ou à 60°C ou à 55°C ou à 50°C.

De préférence, à l'étape ii), en particulier à l'étape iib) et/ou iic), le dioxyde de carbone est mis sous pression, en particulier maintenu à l'étape iic), à une pression supérieure ou égale à 50 bars, encore de préférence supérieure ou égale à 60 bars, préférentiellement supérieure ou égale à 70 bars ou à 80 bars ou à 90 bars, encore préférentiellement supérieure ou égale à 100 bars ou à 110 bars ou à 120 bars ou à 130 bars ou à 140 bars ou à 150 bars.

De préférence, à l'étape ii), en particulier à l'étape iib) et/ou iic), le dioxyde de carbone est mis sous pression, en particulier maintenu à l'étape iic), à une pression inférieure ou égale à 600 bars, encore de préférence inférieure ou égale à 500 bars, préférentiellement inférieure ou égale à 480 bars ou à 460 bars ou à 440 bars, encore préférentiellement inférieure ou égale à 420 bars ou à 380 bars ou à 360 bars ou à 340 bars ou à 320 bars ou à 300 bars ou à 250 bars ou à 200 bars.

Dans un mode de réalisation, à l'étape ii), en particulier à l'étape iib) et/ou iic), le dioxyde de carbone est mis sous pression à une pression comprise entre 100 bars et 300 bars et chauffé à une température comprise entre 35°C et 50°C.

Dans un mode de réalisation préféré, à l'étape ii), en particulier à l'étape iib) et/ou iic), le dioxyde de carbone est mis sous pression à une pression de 150 bars à +/- 20 bars près, et chauffé à une température de 38°C à +/- 5°C près.

De préférence, la durée de traitement du dispositif implantable à l'étape ii), en particulier à l'étape iic) est supérieure ou égale à 5 minutes, encore de préférence supérieure ou égale à 10 minutes ou à 15 minutes ou à 20 minutes, préférentiellement supérieure ou égale à 25 minutes ou à 30 minutes.

De préférence, la durée de traitement du dispositif implantable à l'étape ii), en particulier à l'étape iic), est inférieure ou égale à 60 minutes, encore de préférence inférieure ou égale à 50 minutes ou à 40 minutes ou à 35 minutes.

Dans un mode de réalisation préféré, la durée de traitement du dispositif implantable à l'étape ii), en particulier à l'étape iic), est d'environ 30 minutes à +/- 5 minutes.

Dans un mode de réalisation préféré, la quantité de dioxyde de carbone dans l'état supercritique alimenté à l'enceinte dans l'étape iic) est comprise entre 500 Kg/m³ et 1200 Kg/m³, de préférence entre 600 Kg/m³ et 1100 Kg/m³, encore de préférence entre 700 Kg/m³ et 900 Kg/m³, préférentiellement de 800 Kg/m³ à +/- 50 Kg/m³ près. Dans un mode de réalisation, le volume de CO₂sc alimenté dans l'enceinte de traitement correspond à au moins 20%, ou à au moins 30% ou à au moins 40%, de préférence à au moins 50%, du volume total de traitement de l'enceinte de traitement, encore de préférence à environ 66% à +/- 10% du volume total de traitement de l'enceinte de traitement.

### Protocole d'hydrolyse

L'hydrolyse d'un textile, et notamment du dispositif implantable inventif ou comparatif, comprend les étapes suivantes :
- un ou plusieurs échantillons du dispositif implantable, notamment un ou plusieurs échantillons textiles, ayant (chacun) des dimensions de 10 cm x 10 cm est/sont introduit(s) dans un récipient en verre comprenant 10 litres d'eau purifiée puis tamponnée avec une solution saline tamponnée au phosphate (PBS) afin d'obtenir un pH de 7.4. Le récipient dans un matériau inerte, en particulier en verre, et fermé hermétiquement avec un couvercle évitant ainsi la perte d'eau par évaporation (le volume de 10 litres reste donc constant). Ce récipient est ensuite introduit dans une étuve à une température de 37°C. Une agitation manuelle est réalisée tous les jours pendant environ 15 secondes afin d'uniformiser le milieu d'hydrolyse. En particulier, entre chaque agitation manuelle, aucune agitation mécanique supplémentaire n'est réalisée.

L'échantillon dont on souhaite analyser la résistance mécanique au terme d'une durée de n mois, ou n*4 semaines, d'hydrolyse est extrait du récipient, puis séché, en particulier dans une étuve à 37°C pendant 60 minutes. La durée d'hydrolyse correspond ainsi au temps pendant lequel l'échantillon est resté dans le volume de PBS et disposé en étuve à 37°C.

On comprend dans le présent texte par n mois, une durée équivalente en semaines à n * 4, n étant un nombre entier.

Dans une variante de réalisation, au moins 80% en masse, de préférence au moins 85% ou au moins 87% ou au moins 89% ou au moins 91% ou au moins 93% ou au moins 95% ou au moins 97% ou au moins 99% ou environ 100% en masse, de la masse totale du dispositif implantable fourni à l'étape i) est résorbable, en particulier biorésorbable.

De préférence, au moins 85% ou au moins 87% ou au moins 89% ou au moins 91% ou au moins 93% ou au moins 95% ou au moins 97% ou au moins 99% ou environ 100% en masse, de la masse totale du dispositif implantable et/ou d'un textile (que comprend le dispositif implantable) fourni à l'étape i) est formé d'un ou plusieurs (co)polymère(s) choisi(s) parmi :
- un polymère d'acide lactique de forme L ou D ou de formes L et D, d'un ou plusieurs polymère(s) d'acide glycolique, d'un ou plusieurs copolymère(s) d'acide lactique (L et/ou D) et d'acide glycolique, ou un mélange de ces derniers, de préférence d'un ou plusieurs polymère(s) d'acide lactique de forme L ou D ou de formes L et D (liste I) ; et/ou
- est formé d'un ou plusieurs monofilament(s) chacun comprenant un ou plusieurs (co)polymère(s) choisi(s) dans la liste I ci-dessus.

Dans une variante de réalisation, ledit procédé comprend une étape iv) de stérilisation du dispositif implantable effectuée après l'étape iii), en particulier comprenant l'application d'oxyde d'éthylène audit dispositif implantable.

Avantageusement, toute méthode de stérilisation connue de l'homme du métier peut être utilisée.

Dans une variante de réalisation, le dispositif implantable de l'étape iii) a une résistance mécanique, ou valeur d'éclatement (Newtons/cm), supérieure ou égale à 10 N/cm après 32 semaines ou 8 mois d'hydrolyse, en particulier dans un milieu aqueux tamponné avec une solution saline de phosphate à un pH de 7.4 et maintenu à une température de 37°C dans un milieu fermé hermétiquement.

Dans une variante de réalisation, la température du CO₂ supercritique lors de l'étape ii), en particulier lors de l'étape iib) et/ou iic), est inférieure ou égale à 70°C, en particulier supérieure ou égale à 10°C.

Dans une variante de réalisation, l'étape de traitement ii), en particulier lors de l'étape iib) et/ou iic), comprend l'application de CO₂ supercritique à une pression supérieure ou égale à 50 bars, en particulier inférieure ou égale à 400 bars.

Avantageusement, la température et la pression appliquées ne modifient pas les propriétés mécaniques du dispositif implantable.

Dans une variante de réalisation, l'étape de traitement ii), en particulier lors de l'étape iib) et/ou de l'étape iic), comprend l'application de CO₂ supercritique à une pression supérieure ou égale à 100 bars.

Dans une variante de réalisation, le dispositif implantable fourni à l'étape i) comprend un ou de fil(s) monofilamentaire(s) comprenant chacun au moins un (co)polymère d'acide lactique, notamment un polymère d'acide lactique de forme L ou de forme D, ou une combinaison de ces dernières.

Dans une variante de réalisation, le ou lesdits fil(s) monofilamentaire(s) comprenant chacun au moins un (co)polymère d'acide lactique a, ou ont chacun, un diamètre supérieur ou égal à 50 µm (soit 0,050 mm) et inférieur ou égale à 500 µm (soit 0,50 mm), en particulier inférieur ou égal à 300 µm (soit 0,30 mm).

Dans une variante de réalisation, ledit au moins un (co)polymère d'acide lactique a une température de transition vitreuse (Tg) supérieure ou égale à 40°C et inférieure ou égale à 150°C.

De préférence, ledit au moins un (co)polymère d'acide lactique a une température de transition vitreuse (Tg) supérieure ou égale à 50°C, encore de préférence supérieure ou égale à 60°C, préférentiellement supérieure ou égale à 70°C, encore plus préférentiellement supérieure ou égale à 80°C.

De préférence, ledit au moins un (co)polymère d'acide lactique a une température de transition vitreuse (Tg) inférieure ou égale à 120°C, encore de préférence inférieure ou égale à 110°C, préférentiellement inférieure ou égale à 100°C, encore plus préférentiellement inférieure ou égale à 95°C.

Dans une variante de réalisation, ledit au moins un (co)polymère d'acide lactique a une température de fusion (Tf) supérieure ou égale à 130°C et inférieure ou égale à 210°C.

De préférence, ledit au moins un (co)polymère d'acide lactique a une température de fusion (Tf) supérieure ou égale à 140°C, encore de préférence supérieure ou égale à 150°C, préférentiellement supérieure ou égale à 160°C, encore préférentiellement supérieure ou égale à 165°C.

De préférence, ledit au moins un (co)polymère d'acide lactique a une température de fusion (Tf) inférieure ou égale à 200°C, encore de préférence inférieure ou égale à 190°C, préférentiellement inférieure ou égale à 180°C.

De préférence, ledit au moins un (co)polymère d'acide lactique a un taux de cristallinité supérieur ou égal à 30%, ou à 40% ou à 45%, encore de préférence supérieur ou égal à 50% ou à 53%.

De préférence, ledit au moins un (co)polymère d'acide lactique a un taux de cristallinité inférieur ou égal à 80%, ou à 70% ou à 65%, encore de préférence inférieur ou égal à 60%.

Avantageusement, on comprend par taux de cristallinité le rapport de la somme totale des fractions cristallines en masse ou en volume d'un échantillon donné par rapport à la masse ou au volume total(e) dudit échantillon.

Avantageusement, la température de transition vitreuse, la température de fusion, et le taux de cristallinité, sont déterminées sur le fil monofilamentaire comprenant ledit (co)polymère d'acide lactique.

De préférence, les températures de fusion et de transition vitreuse, et le taux de cristallinité, sont mesurées à l'aide des normes suivantes : ISO 11357-2 (2013) intitulée « Détermination de la température de transition vitreuse et de la hauteur de palier de transition vitreuse », ISO 11357-3 (2018) intitulée « Détermination de la température et de l'enthalpie de fusion et de cristallisation.

L'appareillage est un DSC à compensation de puissance, DSC Q2000 (TA Instruments). Les conditions opératoires sont de préférence les suivantes : le mode de l'appareil est standard, les creusets porte-échantillons sont en aluminium hermétiques, le gaz de balayage est de l'azote de qualité U (50 ml/min), la rampe de température est isotherme à 25°C pendant 5 minutes, puis augmente de 25°C à 250°C avec un gradient de 10°C par minute. Les éprouvettes sont maintenues deux heures à température ambiante (23°C) et 50% d'humidité relative à +/- 10% près.

Dans une variante de réalisation, ledit au moins un (co)polymère d'acide lactique a une masse molaire moyenne en poids Mw supérieure ou égale à 70 000 g/mole et inférieure ou égale à 300 000 g/mole.

De préférence, ledit au moins un (co)polymère d'acide lactique a une masse molaire moyenne en poids Mw supérieure ou égale à 80 000 g/mole, ou à 90 000 g/mole, ou à 100 000 g/mole, encore de préférence supérieure ou égale à 110 000 g/mole, ou à 120 000 g/mole, ou à 130 000 g/mole, ou à 140 000 g/mole, ou à 145 000 g/mole. De préférence, ledit au moins un (co)polymère d'acide lactique a une masse molaire moyenne en poids Mw inférieure ou égale à 250 000 g/mole, ou à 230 000 g/mole, ou à 210 000 g/mole, encore de préférence inférieure ou égale à 200 000 g/mole, ou à 190 000 g/mole, ou à 180 000 g/mole, ou à 170 000 g/mole, ou à 160 000 g/mole.

Dans une variante de réalisation, ledit au moins un (co)polymère d'acide lactique a une masse molaire moyenne en nombre Mn supérieure ou égale à 10 000 g/mole et inférieure ou égale à 120 000 g/mole ou à 110 000 g/mole.

De préférence, ledit au moins un (co)polymère d'acide lactique a une masse molaire moyenne en nombre Mn supérieure ou égale à 20 000 g/mole, ou à 30 000 g/mole, encore de préférence supérieure ou égale à 40 000 g/mole, ou à 45 000 g/mole.

De préférence, ledit au moins un (co)polymère d'acide lactique a une masse molaire moyenne nombre Mn inférieure ou égale à 90 000 g/mole, ou à 80 000 g/mole, encore de préférence inférieure ou égale à 70 000 g/mole, ou à 60 000 g/mole.

Dans une variante de réalisation, ledit au moins un (co)polymère d'acide lactique a une polydispersité Ip (Mw/Mn) supérieure ou égale à 1,5 ou à 2 et inférieure ou égale à 5, de préférence inférieure ou égale à 4, encore de préférence inférieure ou égale à 3,5 ou 3.

Avantageusement, les masses molaires moyennes en poids et en nombre (g/mole) sont déterminées sur le fil monofilamentaire comprenant ledit (co)polymère d'acide lactique.

De préférence, les masses molaires moyennes en nombre et en poids sont mesurées à l'aide de la norme ASTM D3536-91 intitulée « Méthode d'essai normalisée pour les masses moléculaires moyennes en poids et leur distribution par chromatographie d'exclusion en phase liquide (Chromatographie par perméation de gel- GPC) ». Cette technique est également appelée chromatographie d'exclusion stérique.

De préférence, les conditions opératoires sont les suivantes : systèmes GPC Agilent 1260 Infinity, Système 4 colonnes Waters Styragel, 300*4.6 mm, particules de 5 µm, de porosité de 50 à 104 A, ensemble thermostaté à 40°C, un éluant THF qualité analyse filtré avec un débit de 0,5 ml/min, une préfiltration des échantillons sur filtre PTFE 0,2 µm, une double détection RI et UV (254 nm), un étalonnage polystyrène, préparation de l'échantillon par dissolution dans un mélange tétrahydrofurane (THF)/Toluène (utilisé comme marqueur).

Dans une variante de réalisation, ledit au moins un (co)polymère d'acide lactique comprend des unités d'acide lactique de forme L et des unités d'acide lactique de forme D.

Avantageusement, les formes L et D ainsi que leurs proportions sont déterminées sur le fil monofilamentaire comprenant ledit (co)polymère d'acide lactique.

De préférence, pour déterminer le pouvoir rotatoire, les échantillons à tester sont mis en solution dans le chloroforme à une concentration de 1g/dl.

De préférence, le monomère de forme D ou L est dosé par chromatographie en phase gazeuse couplée avec la spectrométrie de masse.

De préférence, les conditions opératoires sont les suivantes : chaque échantillon est extrait dans le dichlorométhane, le polymère est précipité dans l'hexane, et le mélange filtré sur filtre seringue 0,2µm PTFE, le dosage est réalisé par étalonnage externe (monomère lactide forme D ou L) à l'aide de quatre standards préparés à différentes concentrations ; les conditions chromatographiques sont de préférence les suivantes : spectromètre Agilent HP5975C, GC 7890 Agilent, Colonne HP-5MS ( 5% polyphénylsilowane) 30m, épaisseur 0,25 µm, diamètre 0,25 mm, isotherme de 1 min à 50°C puis chauffage à 25°C/min de 50°C à 320°C, isotherme de 5min à 320°C, l'injecteur est chauffé à 200°C, l'injection de 2µl en mode splitless, détection en mode SCAN.

Dans une variante de réalisation, ledit au moins un (co)polymère d'acide lactique comprend au moins 80% en masse d'unités d'acide lactique de forme L et moins de 20% en masse d'unités d'acide lactique de forme D.

De préférence, ledit au moins un (co)polymère d'acide lactique comprend au moins 85% ou 88% ou 90% ou 92% ou 94% ou 96% ou 98% en masse d'unités d'acide lactique de forme L.

De préférence, ledit au moins un (co)polymère d'acide lactique comprend au plus 15% ou au plus 10% ou au plus 8% ou au plus 6% en masse d'unités d'acide lactique de forme D, encore de préférence au plus 5% ou 4% en masse d'unités d'acide lactique de forme D.

De préférence, ledit au moins un (co)polymère d'acide lactique comprend au moins 0,5% ou 1% ou 1,5% en masse d'unités d'acide lactique de forme D.

Dans une variante de réalisation, au moins 80%, de préférence au moins 85% ou 90% ou 95%, en masse du dispositif implantable comprend un ou des fil(s) monofilamentaire(s) dans un ou plusieurs (co)polymère(s) d'acide lactique.

L'amélioration de la résistance mécanique, ou valeur d'éclatement, à l'hydrolyse est significative à partir de 3 mois d'hydrolyse pour un dispositif implantable comprenant des fils monofilamentaires en (co)polymère d'acide lactique, tricotées à mailles jetées, et en particulier avec un polymère d'acide lactique tel que décrit ci-dessus.

Dans une variante de réalisation, ledit procédé comprend une étape de thermofixation du ou des textile(s) (que comprend le dispositif implantable) ou du dispositif implantable, de préférence ayant lieu avant l'étape ii) ou après l'étape ii).

De préférence, ladite étape de thermofixation comprend l'application d'une température supérieure ou égale à 80°C et inférieure ou égale à 130°C, encore de préférence supérieure ou égale à 90°C et inférieure ou égale à 120°C, préférentiellement de 110°C à +/- 5°C.

De préférence, la durée de ladite étape de thermofixation est supérieure ou égale à 30 secondes, encore de préférence supérieure ou égale à 60 secondes, préférentiellement d'environ 120 secondes à +/- 30 secondes.

De préférence, la durée de ladite étape de thermofixation est inférieure ou égale à 2 heures, encore de préférence inférieure ou égale à 1 heure, préférentiellement inférieure ou égale à 30 min ou à 20 min ou à 10 min ou à 5 min.

De préférence, aucune force de traction n'est exercée sur le ou les tricots ou le dispositif implantable durant l'étape de thermofixation, en particulier le ou les tricots ou le dispositif est/sont libre(s) de tension.

La présente invention a pour objet, selon second aspect, un dispositif implantable, en particulier pour le traitement d'une hernie abdominale, en particulier ventrale ou inguinale, susceptible d'être obtenu par le procédé selon l'une quelconque des variantes de réalisation en référence au premier aspect de l'invention et/ou tel que décrit dans le présent texte.

Avantageusement, le dispositif implantable obtenu a une résistance mécanique ou valeur d'éclatement améliorée au terme de trois à huit mois d'hydrolyse comparativement un dispositif similaire non traité par du CO₂ sc.

Dans une variante de réalisation, au moins 80% en masse, de préférence au moins 85% ou 90% ou 95% ou 98% en masse, du dispositif implantable est formée d'un ou plusieurs fil(s) monofilamentaire(s) dans un ou plusieurs (co)polymère(s) d'acide lactique, et le dispositif implantable a une résistance mécanique supérieure ou égale à 10N/cm, de préférence supérieure ou égale à 15N/cm ou à 20N/cm, encore de préférence 25 N/cm, au terme de 20 semaines d'hydrolyse.

Les variantes et définitions selon le premier aspect de l'invention s'appliquent indépendamment les unes des autres au dispositif implantable selon un second aspect de l'invention.

La présente invention a pour objet, selon un troisième aspect, l'utilisation de CO₂ supercritique pour améliorer la résistance mécanique (N/cm), ou valeur d'éclatement (N/cm), d'un dispositif implantable comprenant un ou des fil(s) comprenant au moins un (co)polymère d'acide lactique au terme d'une période d'hydrolyse déterminée, notamment en milieu aqueux déterminée, en particulier au terme d'une période d'au moins 20 semaines pour obtenir une résistance mécanique supérieure ou égale à 10 N/cm, de préférence supérieure ou égale à 15N/cm ou 20N/cm, encore de préférence supérieure ou égale à 25 N/cm.

En particulier, ladite utilisation comprend l'application de CO₂ supercritique audit dispositif implantable et/ou au(x) dit(s) fil(s) monofilamentaire(s).

Les variantes et définitions selon le premier aspect de l'invention et/ou le second aspect de l'invention s'appliquent indépendamment les unes des autres au troisième aspect de l'invention.

Le présent texte comprend également selon un quatrième aspect une méthode d'utilisation d'un dispositif implantable comprenant :
- la mise en œuvre du procédé pour induire une amélioration de la résistance mécanique d'un dispositif implantable comprenant un ou plusieurs fil(s) comprenant (chacun) un ou plusieurs (co)polymère(s) d'acide lactique selon l'une quelconque des variantes de réalisation en référence au premier aspect de l'invention, ou la fourniture d'un dispositif implantable selon un second aspect de l'invention ou susceptible d'être obtenu par ledit procédé pour induire une amélioration de la résistance mécanique selon l'une quelconque des variantes de réalisation en référence au premier aspect de l'invention, et
- la sélection d'un groupe de patients choisi parmi les groupes suivants : les patients ayant un IMC supérieur ou égal à 30, les patients souffrant de diabète de type I et/ou II, les patients fumeurs (ou souffrant de tabagisme), et les patients choisis parmi une combinaison desdits groupes de patients ;
- le traitement du groupe de patient sélectionné pour le traitement d'une hernie abdominale, en particulier ventrale ou inguinale, avec ledit dispositif implantable. L'IMC est l'acronyme de « Indice de Masse Corporelle » calculé en divisant le poids (Kg) par la taille (cm) au carré.

### Description des dessins

La présente invention sera mieux comprise à la lecture des modes de réalisation suivants, cités à titre non limitatif, et illustrés par les figures dans lesquelles :
[Fig.1] la figure 1 représente schématiquement les différents états (solide, liquide, gazeux, supercritique) du dioxyde de carbone en fonction de la pression (bars) en ordonnée et de la température (°C) en abscisse ;
[Fig.2] la figure 2 est un tableau reprenant les valeurs de résistance mécanique (valeur d'éclatement à la bille) pour un exemple selon l'invention (EX1) et deux exemples comparatifs (EXC1, EXC2) sans hydrolyse et pour différentes périodes d'hydrolyse : 1 mois, 2 mois, 3 mois, 5 mois, et 8 mois ;
[Fig.3] la figure 3 est un tableau reprenant les résistances mécaniques (valeurs d'éclatement à la bille) résiduelles mesurées en rapportant la résistance mécanique à T1,2,3,5, ou 8 mois sur la résistance mécanique initiale T0 multipliée par 100, lesdites résistances mécaniques étant indiquées à la figure 2;
[Fig.4] la figure 4 est un graphique représentant en ordonnée les valeurs d'éclatement à la bille résiduelles (%) en ordonnée en fonction de la durée d'hydrolyse en abscisse de plusieurs exemples de dispositif implantable selon l'invention et d'exemples comparatifs de dispositif implantables ;
[Fig.5] la figure 5 est un graphique représentant en ordonnée les valeurs d'éclatement à la bille (N/cm) en ordonnée en fonction de la durée d'hydrolyse en abscisse de plusieurs exemples de dispositif implantable selon l'invention et d'exemples comparatifs de dispositif implantables ;
[Fig.6] la figure 6 représente le schéma de mailles du premier exemple de dispositif implantable selon l'invention et des exemples comparatifs de dispositif implantable.

### Description des modes de réalisation

Le diagramme de la figure 1 représente les différents états : solide, liquide, gazeux et supercritique du dioxyde de carbone selon les conditions de pression (bars) et de température qui lui sont appliquées.

Ainsi, on observe un point dit triple à -56°C et 5,18 bars à l'intersection duquel on observe les frontières entre les états solide, liquide et gazeux.

Le point critique à partir duquel, le dioxyde de carbone est dans un état supercritique comprend une température supérieure ou égale à 31°C combinée avec une pression supérieure ou égale à 73,85 bars. On observe que dans cet état supercritique, le dioxyde de carbone est à la fois dans des conditions proches de l'état liquide ce qui lui permet de se comporter comme un solvant, dans des conditions entre un gaz et un liquide ce qui lui permet de venir imprégner le dispositif implantable et dans des conditions proches de l'état gazeux ce qui lui permettrait de diffuser dans la structure des fils monofilamentaires à base d'acide lactique. Le Demandeur a observé de façon surprenante qu'un traitement par du CO₂sc, en particulier de la structure textile du dispositif implantable, permet d'améliorer significativement la résistance mécanique du dispositif implantable durant son hydrolyse.

Avantageusement, le traitement par du CO₂sc selon l'invention permet d'améliorer le comportement mécanique du dispositif implantable à l'hydrolyse, en particulier de réduire la vitesse d'hydrolyse, mais n'est pas un traitement en vue de sa stérilisation. Dans un mode de réalisation, une étape de stérilisation, distincte de l'étape de traitement par du CO₂sc pour induire une amélioration de la résistance mécanique est nécessaire. Cette étape de stérilisation peut être effectuée par toute méthode de stérilisation connue dans le domaine technique pour stériliser un dispositif implantable, en particulier déjà disposé dans un double sachet.

Les figures 2 à 5 représentent les résultats des tests à l'hydrolyse obtenus pour un exemple EX1 de réalisation selon l'invention, et deux exemples comparatifs EXC1 et EXC2.

L'exemple EX1 est un tricot 10 à mailles jetées tricoté selon le schéma de mailles représenté à la figure 6. Ce tricot comprend un premier fil monofilamentaire 20 dans un polymère d'acide lactique ayant un diamètre de 0,15 mm et une masse linéique de 220 dtex, et un second fil 30 monofilamentaire similaire au premier fil monofilamentaire (dans un polymère d'acide lactique ayant un diamètre de 0,15 mm et une masse linéique de 220 dtex). Le premier fil 20 est supporté par une première barre de guidage B1 sur le métier à tricoter dont le mouvement est le suivant : 2- 3/ 2- 1/ 2- 3/ 2- 1/ 1- 0/ 1- 2/ 1- 0/ 1- 2//.

Le second fil 30 est supporté par une seconde barre de guidage B2 sur le métier à tricoter dont le mouvement est le suivant : 1- 0/ 1- 2/ 1- 0/ 1- 2/ 2- 3/ 2- 1/ 2- 3/ 2-1//.

Le tricot 10 a une masse surfacique d'environ 120 g/m², et est totalement résorbable. Les première et seconde barres de guidage, ou dites à passettes, B1, B2 travaillent/tricotent sur toute la largeur du tricot 10 et sur toute sa hauteur.

Avantageusement, les première et secondes barres de guidage B1, B2 travaillent en opposition : la première barre de guidage supportant le fil 20 travaille dans la direction F2 lorsque la seconde barre de guidage supportant le fil 30 travaille dans la direction F1, les directions F1 et F2 étant opposées comme représentées sur la figure 6. Dans cet exemple précis, le tricot 10 subit une étape de thermofixation à 110°C pendant 2 min, puis subit un traitement au CO₂sc consistant à le placer dans un autoclave à 38°C, sous une pression de 150 bars pendant 30 min afin de maintenir le CO₂ dans son état supercritique. Le volume de CO₂sc alimenté dans l'enceinte de traitement représente environ 66% du volume total de traitement de l'enceinte.

Les exemples comparatifs EXC1 et EXC2 sont chacun dans un tricot identique au tricot 10 mais ne subissent aucun traitement au CO₂sc. L'EXC2 subit une étape de thermofixation à 110°C pendant 2 minutes similaire à l'étape de thermofixation de l'EX1 tandis que l'EXC1 ne subit pas d'étape de thermofixation.

Pour la réalisation des tests d'hydrolyse : on introduit 30 échantillons de tricot 10cm * 10cm dans un récipient en inox comprenant de l'eau tamponnée au PBS de chaque exemple (EX1, EXC1, EXC2) selon le protocole d'hydrolyse décrit ci-avant. Au terme de chaque période d'hydrolyse visée (1 mois, 2 mois, 3 mois, 5 mois, 8 mois), 18 échantillons (6 échantillons de chaque exemple) sont retirés du récipient inox puis séchés en étuve à 37°C pendant une heure. Ces 18 échantillons sont tous évalués au test de valeur d'éclatement décrit ci-avant (ASTM D3787-7 (2011)) immédiatement après séchage.

La résistance mécanique évaluée est ici avantageusement une valeur d'éclatement à la bille. Il pourrait s'agir d'une autre valeur de résistance mécanique, telle que la charge à rupture dans le sens chaine ou trame du tricot, i.e. toute mesure permettant d'évaluer l'améliorer de la résistance mécanique du tricot.

La température de fusion et le taux de cristallinité mesurés pour l'EX1 à T0 sont respectivement de 169,53°C et de 59%, et sont à T1 (mois) de 167,88°C et 46%.

La température de fusion et le taux de cristallinité mesurés pour l'EXC1 à T0 sont respectivement de 166,76°C et de 56%, et sont à T1 (mois) de 166,83°C et 56%.

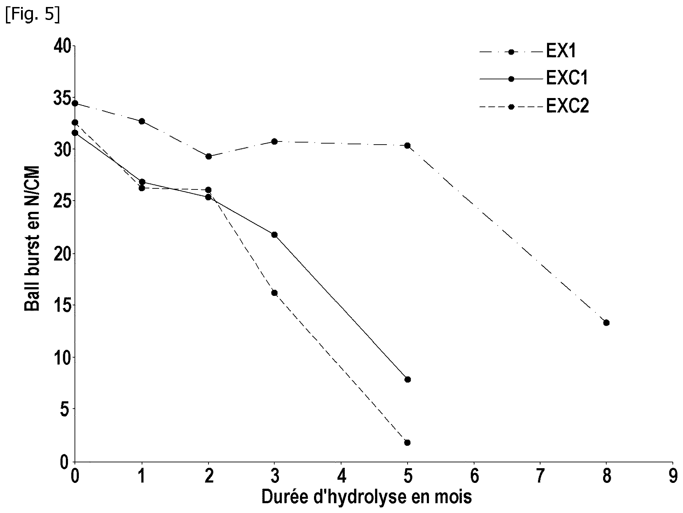

De manière surprenante, on observe sur les figures 4 et 5 déjà qu'au terme de 2 mois d'hydrolyse, la résistance mécanique de l'EX1 selon l'invention est dégradée mais se maintient à plus de 80% de la résistance mécanique initiale alors que celles des exemples EXC1 et EXC2 commencent à baisser de manière importante. Au terme de trois mois et jusqu'à environ 5 mois la résistance mécanique est encore au moins de plus de 80% de la résistance mécanique initiale pour l'EX1 alors qu'elle chute de plus de 40% à trois mois et à plus de 75% à 5 mois de la résistance initiale pour les exemples EXC1 et EXC2. Enfin, au terme de 8 mois d'hydrolyse, le tricot selon l'EX1 présente encore une résistance mécanique correspondant à environ 40% de sa résistance initiale tandis que les tricots des exemples EXC1 et EXC2 sont totalement hydrolysés ou trop hydrolysés pour qu'une valeur de résistance puisse être mesurée. L'utilisation du CO₂sc pour améliorer la résistance mécanique d'un textile comprenant des monofilaments dans un (co)polymère d'acide lactique permet d'améliorer le comportement à l'hydrolyse de ce dernier, en particulier de réduire sa vitesse d'hydrolyse, de manière simple et fiable à partir d'une structure textile résorbable fiable dans le traitement d'une hernie par exemple. Cette disposition permettra ainsi de prolonger le renfort mécanique apportée par le dispositif implantable, tout en limitant les risques d'infection, et évitera de recourir à une chirurgie de retrait du dispositif implantable en cas d'infection puisque le dispositif finira par se résorber totalement.

## Revendications

1. Procédé pour induire une amélioration de la résistance mécanique (N/cm) d'un dispositif implantable (10) après une période déterminée d'hydrolyse dudit dispositif implantable (10), ledit dispositif implantable (10) comprenant un ou des fil(s) (20,30) comprenant au moins un (co)polymère d'acide lactique, ledit procédé comprenant :
i)- une étape de fourniture d'un dispositif implantable (10) comprenant un ou des fil(s) (20,30) comprenant au moins un (co)polymère d'acide lactique;
ii)- une étape de traitement dudit dispositif implantable avec du dioxyde de carbone (CO₂) supercritique ;
iii)- une étape d'obtention d'un dispositif implantable (10) ayant une résistance mécanique supérieure ou égale à 10 N/cm, de préférence supérieure ou égale à 25 N/cm, après 20 semaines d'hydrolyse, en particulier dans un milieu aqueux.

2. Procédé selon la revendication 1, **caractérisé en ce qu'**au moins 80% en masse de la masse totale du dispositif implantable (10) fourni à l'étape i) est résorbable.

3. Procédé selon l'une l'autre des revendications 1 et 2, **caractérisé en ce qu'**il comprend une étape iv) de stérilisation du dispositif implantable (10) effectuée après l'étape iii), en particulier comprenant l'application d'oxyde d'éthylène audit dispositif implantable (10).

4. Procédé selon l'une quelconque des revendications 1 à 3, caractérisé en ce le dispositif implantable (10) de l'étape iii) a une résistance mécanique supérieure ou égale à 10 N/cm après 32 semaines d'hydrolyse dans un milieu aqueux.

5. Procédé selon l'une quelconque des revendications 1 à 4, **caractérisé en ce que** la température du CO₂ supercritique lors de l'étape ii) est inférieure ou égale à 70°C, en particulier supérieure ou égale à 10°C.

6. Procédé selon l'une quelconque des revendications 1 à 5, **caractérisé en ce que** l'étape de traitement ii) comprend l'application de CO₂ supercritique à une pression supérieure ou égale à 10 bars, en particulier inférieure ou égale à 500 bars.

7. Procédé selon l'une quelconque des revendications 1 à 6, **caractérisé en ce que** l'étape de traitement ii) comprend l'application de CO₂ supercritique à une pression supérieure ou égale à 100 bars.

8. Procédé selon l'une quelconque des revendications 1 à 7, **caractérisé en ce que** le dispositif implantable (10) fourni à l'étape i) comprend un ou de fil(s) monofilamentaire(s) (20,30) comprenant chacun au moins un (co)polymère d'acide lactique.

9. Procédé selon la revendication 8, **caractérisé en ce que** le ou lesdits fil(s) monofilamentaire(s) (20,30) comprenant chacun au moins un (co)polymère d'acide lactique a, ou ont chacun, un diamètre supérieur ou égal à 50 µm et inférieur ou égale à 500 µm, en particulier inférieur ou égal à 300 µm.

10. Procédé selon l'une quelconque des revendications 1 à 9, **caractérisé en ce que** ledit au moins un (co)polymère d'acide lactique a une température de transition vitreuse (Tg) supérieure ou égale à 40°C et inférieure ou égale à 150°C.

11. Procédé selon l'une quelconque des revendications 1 à 10, **caractérisé en ce que** ledit au moins un (co)polymère d'acide lactique a une température de fusion (Tf) supérieure ou égale à 130°C et inférieure ou égale à 210°C.

12. Procédé selon l'une quelconque des revendications 1 à 11, **caractérisé en ce que** ledit au moins un (co)polymère d'acide lactique a une masse molaire moyenne en poids Mw supérieure ou égale à 70 000 g/mole et inférieure ou égale à 300 000 g/mole.

13. Procédé selon l'une quelconque des revendications 1 à 12, **caractérisé en ce que** ledit au moins un (co)polymère d'acide lactique a une polydispersité Ip (Mw/Mn) supérieure ou égale à 1,5 et inférieure ou égale à 5.

14. Procédé selon l'une quelconque des revendications 1 à 13, **caractérisé en ce que** ledit au moins un (co)polymère d'acide lactique comprend des unités d'acide lactique de forme L et des unités d'acide lactique de forme D.

15. Procédé selon l'une quelconque des revendications 1 à 14, **caractérisé en ce que** ledit au moins un (co)polymère d'acide lactique comprend au moins 80% en masse d'unités d'acide lactique de forme L et moins de 20% en masse d'unités d'acide lactique de forme D.

16. Procédé selon l'une quelconque des revendications 1 à 15, **caractérisé en ce qu'**au moins 80% en masse du dispositif implantable (10) comprend un ou des fil(s) monofilamentaire(s) (20,30) dans un ou plusieurs (co)polymère(s) d'acide lactique.

17. Dispositif implantable, en particulier pour le traitement d'une hernie abdominale (10), susceptible d'être obtenu par le procédé selon l'une quelconque des revendications 1 à 16.

18. Dispositif implantable (10) selon la revendication 17, **caractérisé en ce qu'**il comprend au moins 80% en masse d'un ou plusieurs fil(s) monofilamentaire(s) (20,30) dans un ou plusieurs (co)polymère(s) d'acide lactique, et **en ce que** le dispositif implantable (10) a une résistance mécanique supérieure ou égale à 25 N/cm au terme de 20 semaines d'hydrolyse.

19. Utilisation du CO₂ supercritique pour améliorer la résistance mécanique (N/cm) d'un dispositif implantable (10) comprenant un ou des fil(s) (20,30) comprenant au moins un (co)polymère d'acide lactique au terme d'une période d'hydrolyse déterminée, en particulier au terme d'une période d'hydrolyse d'au moins 20 semaines pour obtenir une résistance mécanique supérieure ou égale à 10 N/cm, en particulier supérieure ou égale à 25 N/cm.

## Patentansprüche

1. Verfahren zum Induzieren einer Verbesserung der mechanischen Festigkeit (N/cm) einer implantierbaren Vorrichtung (10) nach einer bestimmten Hydrolysezeit der implantierbaren Vorrichtung (10), wobei die implantierbare Vorrichtung (10) einen oder mehrere Fäden (20, 30) umfasst, umfassend mindestens ein Milchsäure-(co-)polymer, das Verfahren umfassend:
i) - einen Schritt eines Bereitstellens einer implantierbaren Vorrichtung (10), umfassend einen oder mehrere Fäden (20, 30), umfassend mindestens ein Milchsäure-(co-)polymer;
ii) - einen Schritt eines Behandelns der implantierbaren Vorrichtung mit überkritischem Kohlendioxid (CO₂);
iii) - einen Schritt eines Erlangens einer implantierbaren Vorrichtung (10), die eine mechanische Festigkeit von mindestens 10 N/cm, vorzugsweise mindestens 25 N/cm, nach 20 Wochen Hydrolyse, insbesondere in einem wässrigen Medium, aufweist.

2. Verfahren nach Anspruch 1, **dadurch gekennzeichnet, dass** mindestens 80 Gewichts-% der Gesamtmasse der in Schritt i) bereitgestellten implantierbaren Vorrichtung (10) resorbierbar ist.

3. Verfahren nach einem der Ansprüche 1 oder 2, **dadurch gekennzeichnet, dass** es einen Schritt iv) einer Sterilisation der implantierbaren Vorrichtung (10) umfasst, der nach Schritt iii) durchgeführt wird, insbesondere umfassend die Anwendung von Ethylenoxid auf die implantierbare Vorrichtung (10).

4. Verfahren nach einem der Ansprüche 1 bis 3, **dadurch gekennzeichnet, dass** die implantierbare Vorrichtung (10) von Schritt iii) nach 32 Wochen Hydrolyse in einem wässrigen Medium eine mechanische Festigkeit von mindestens 10 N/cm aufweist.

5. Verfahren nach einem der Ansprüche 1 bis 4, **dadurch gekennzeichnet, dass** die Temperatur des überkritischen CO₂ bei Schritt ii) kleiner als oder gleich wie 70 °C, insbesondere größer als oder gleich wie 10 °C ist.

6. Verfahren nach einem der Ansprüche 1 bis 5, **dadurch gekennzeichnet, dass** der Behandlungsschritt ii) die Anwendung von überkritischem CO₂ bei einem Druck größer als oder gleich wie 10 bar, insbesondere kleiner als oder gleich wie 500 bar umfasst.

7. Verfahren nach einem der Ansprüche 1 bis 6, **dadurch gekennzeichnet, dass** der Behandlungsschritt ii) die Anwendung von überkritischem CO₂ bei einem Druck größer als oder gleich wie 100 bar umfasst.

8. Verfahren nach einem der Ansprüche 1 bis 7, **dadurch gekennzeichnet, dass** die in Schritt i) bereitgestellte implantierbare Vorrichtung (10) einen oder mehrere Monofilamentfäden (20, 30) umfasst, jeweils umfassend mindestens ein Milchsäure-(co-)polymer.

9. Verfahren nach Anspruch 8, **dadurch gekennzeichnet, dass** der oder die Monofilamentfäden (20, 30), die jeweils mindestens ein Milchsäure-(co-)polymer umfassen, jeweils einen Durchmesser größer als oder gleich wie 50 µm und kleiner als oder gleich wie 500 µm, insbesondere kleiner als oder gleich wie 300 µm, aufweisen.

10. Verfahren nach einem der Ansprüche 1 bis 9, **dadurch gekennzeichnet, dass** das mindestens eine Milchsäure-(co-)polymer eine Glasübergangstemperatur (Tg) größer als oder gleich wie 40 °C und kleiner als oder gleich wie 150 °C aufweist.

11. Verfahren nach einem der Ansprüche 1 bis 10, **dadurch gekennzeichnet, dass** das mindestens eine Milchsäure-(co-)polymer eine Schmelztemperatur (Tf) größer als oder gleich wie 130 °C und kleiner als oder gleich wie 210 °C aufweist.

12. Verfahren nach einem der Ansprüche 1 bis 11, **dadurch gekennzeichnet, dass** das mindestens eine Milchsäure-(co-)polymer eine gewichtsmittlere molare Masse Mw größer als oder gleich wie 70.000 g/mol und kleiner als oder gleich wie 300.000 g/mol aufweist.

13. Verfahren nach einem der Ansprüche 1 bis 12, **dadurch gekennzeichnet, dass** das mindestens eine Milchsäure-(co-)polymer eine Polydispersität Ip (Mw/Mn) größer als oder gleich wie 1,5 und kleiner als oder gleich wie 5 aufweist.

14. Verfahren nach einem der Ansprüche 1 bis 13, **dadurch gekennzeichnet, dass** das mindestens eine Milchsäure-(co-)polymer Einheiten von Milchsäure in L-Form und Einheiten von Milchsäure in D-Form umfasst.

15. Verfahren nach einem der Ansprüche 1 bis 14, **dadurch gekennzeichnet, dass** das mindestens eine Milchsäure-(co-)polymer mindestens 80 Gewichts-% Einheiten von Milchsäure in L-Form und weniger als 20 Gewichts-% Einheiten von Milchsäure in D-Form umfasst.

16. Verfahren nach einem der Ansprüche 1 bis 15, **dadurch gekennzeichnet, dass** mindestens 80 Gewichts-% der implantierbaren Vorrichtung (10) einen oder mehrere Monofilamentfäden (20, 30) in einem oder mehreren Milchsäure-(co-)polymere umfasst.

17. Implantierbare Vorrichtung, insbesondere zur Behandlung eines Bauchwandbruchs (10), das durch das Verfahren nach einem der Ansprüche 1 bis 16 erlangbar ist.

18. Implantierbare Vorrichtung (10) nach Anspruch 17, **dadurch gekennzeichnet, dass** sie mindestens 80 Gewichts-% eines oder mehrerer Monofilamentfäden (20, 30) in einem oder mehreren Milchsäure-(co-)polymere umfasst, und dass die implantierbare Vorrichtung (10) nach 20 Wochen Hydrolyse eine mechanische Festigkeit größer als oder gleich wie 25 N/cm aufweist.

19. Verwendung von überkritischem CO₂ zur Verbesserung der mechanischen Festigkeit (N/cm) einer implantierbaren Vorrichtung (10), umfassend einen oder mehrere Fäden (20, 30), umfassend mindestens ein Milchsäure-(co-)polymer, nach einer bestimmten Hydrolysezeit, insbesondere nach einer Hydrolysezeit von mindestens 20 Wochen, um eine mechanische Festigkeit größer als oder gleich wie 10 N/cm, insbesondere größer als oder gleich wie 25 N/cm, zu erlangen.

## Claims

1. A method for inducing an improvement in the mechanical strength (N/cm) of an implantable device (10) after a determined period of hydrolysis of said implantable device (10), said implantable device (10) comprising one or more yarn(s) (20, 30) comprising at least one lactic acid (co)polymer, said method comprising:
i) - a step of providing an implantable device (10) comprising one or more yarn(s) (20, 30) comprising at least one lactic acid (co)polymer;
ii) - a step of treating said implantable device with supercritical carbon dioxide (CO₂);
iii) - a step of obtaining an implantable device (10) having a mechanical strength greater than or equal to 10 N/cm, preferably greater than or equal to 25 N/cm, after 20 weeks of hydrolysis, in particular in an aqueous medium.

2. The method according to claim 1, **characterised in that** at least 80% by mass of the total mass of the implantable device (10) provided in step i) is resorbable.

3. The method according to one or other of claims 1 and 2, **characterised in that** it comprises a step iv) of sterilisation of the implantable device (10) performed after step iii), in particular comprising the application of ethylene oxide to said implantable device (10).

4. The method according to any one of claims 1 to 3, **characterised in that** the implantable device (10) of step iii) has a mechanical strength greater than or equal to 10 N/cm after 32 weeks of hydrolysis in an aqueous medium.

5. The method according to any one of claims 1 to 4, **characterised in that** the temperature of the supercritical CO₂ during step ii) is less than or equal to 70°C, in particular greater than or equal to 10°C.

6. The method according to any one of claims 1 to 5, **characterised in that** treatment step ii) comprises the application of supercritical CO₂ at a pressure greater than or equal to 10 bar, in particular less than or equal to 500 bar.

7. The method according to any one of claims 1 to 6, **characterised in that** the treatment step ii) comprises the application of supercritical CO₂ at a pressure greater than or equal to 100 bar.

8. The method according to any one of claims 1 to 7, **characterised in that** the implantable device (10) provided in step i) comprises one or more monofilamentary yarn(s) (20, 30) each comprising at least one lactic acid (co)polymer.

9. The method according to claim 8, **characterised in that** said one or more monofilamentary yarn(s) (20, 30) each comprising at least one lactic acid (co)polymer has, or each have, a diameter greater than or equal to 50 µm and less than or equal to 500 µm, in particular less than or equal to 300 µm.

10. The method according to any one of claims 1 to 9, **characterised in that** said at least one lactic acid (co)polymer has a glass transition temperature (Tg) greater than or equal to 40°C and less than or equal to 150°C.

11. The method according to any one of claims 1 to 10, **characterised in that** said at least one lactic acid (co)polymer has a melting point (Tf) greater than or equal to 130°C and less than or equal to 210°C.

12. The method according to any one of claims 1 to 11, **characterised in that** said at least one lactic acid (co)polymer has a mass average molar mass Mw greater than or equal to 70,000 g/mole and less than or equal to 300,000 g/mole.

13. The method according to any one of claims 1 to 12, **characterised in that** said at least one lactic acid (co)polymer has a polydispersity Ip (Mw/Mn) greater than or equal to 1.5 and less than or equal to 5.

14. The method according to any one of claims 1 to 13, **characterised in that** said at least one lactic acid (co)polymer comprises L-form lactic acid units and D-form lactic acid units.

15. The method according to any one of claims 1 to 14, **characterised in that** said at least one lactic acid (co)polymer comprises at least 80% by mass L-form lactic acid units and less than 20% by mass D-form lactic acid units.

16. The method according to any one of claims 1 to 15, **characterised in that** at least 80% by mass of the implantable device (10) comprises one or more monofilamentary yarn(s) (20, 30) in one or more lactic acid (co)polymer(s).

17. An implantable device, in particular for the treatment of an abdominal hernia (10), which can be obtained by the method according to any one of claims 1 to 16.

18. The implantable device (10) according to claim 17, **characterised in that** it comprises at least 80% by mass of one or more monofilamentary yarn(s) (20, 30) in one or more lactic acid (co)polymer(s), and **in that** the implantable device (10) has a mechanical strength greater than or equal to 25 N/cm at the end of 20 weeks of hydrolysis.

19. Use of supercritical CO₂ for improving the mechanical strength (N/cm) of an implantable device (10) comprising one or more yarn(s) (20, 30) comprising at least one lactic acid (co)polymer at the end of a determined period of hydrolysis, in particular at the end of a period of hydrolysis of at least 20 weeks in order to obtain a mechanical strength greater than or equal to 10 N/cm, in particular greater than or equal to 25 N/cm.
